# EUROPEAN PATENT SPECIFICATION

(11) **EP 0 991 388 B1**
(45) Date of publication and mention of the grant of the patent: **21.01.2004**
(21) Application number: 98931653.4
(22) Date of filing: 26.06.1998
(51) Int. Cl.: A61F 13/15, A61F 5/451

(54) **SHAPED SKIN ATTACHMENT MEANS FOR A FAECAL COLLECTOR**
VORGEFORMTE MITTEL ZUR BEFESTIGUNG EINES FÄKALIENBEHÄLTERS AUF DER HAUT
DISPOSITIF DE FIXATION DE FORME ANATOMIQUE POUR UN COLLECTEUR DE MATIERES FECALES

(30) Priority: 28.06.1997 EP 97110604; 28.06.1997 EP 97110602; 28.06.1997 EP 97110603
(43) Date of publication of application: 12.04.2000
(73) Proprietor: THE PROCTER & GAMBLE COMPANY, Cincinnati, Ohio 45202 (US)
(72) Inventor: PALUMBO, Gianfranco, D-61348 Bad Homburg (DE); D'ACCHIOLI, Vincenzo, D-65779 Kelkheim am Taunus (DE)
(74) Representative: Kohol, Sonia
(86) International application number: PCT/US1998/013357
(87) International publication number: WO 1999/000084

(56) References cited:
- EP-A- 0 753 290
- GB-A- 1 092 274
- GB-A- 2 116 849
- US-A- 3 577 989
- PATENT ABSTRACTS OF JAPAN vol. 096, no. 009, 30 September 1996 & JP 08 117261 A (BEPPU YOSHIO), 14 May 1996 cited in the application

## Description

### Field of the Invention

The present invention relates to a body fitting faecal management device. In particular, the present invention is directed towards faecal management devices with improved skin attachment means such that improved body fit and a tight seal is provided between the body of the wearer and the skin, thereby preventing contact between the faecal material and the urinary tract. The devices find particular utility for female wearers of such devices.

### Background of the Invention

Faecal management devices are known articles of manufacture that are designed to be worn principally by incontinence sufferers and infants. Such faecal management devices are attached to the anal region of the wearer and are intended to entrap and immediately contain faecal material and other bodily discharges. As a consequence, these devices are functionally effective in eliminating the problem of smearing on the skin of the wearer; in lessening epidermal irritation; in preventing contamination of articles such as clothing and bedding; and even in preventing the soiling of the carers themselves. Nevertheless, a problem often encountered during the use of such faecal management devices is that some of the faecal material can flow towards the urinary tract of the wearer in particular for female wearers into the sensitive urethra of the wearer. Typically, the presence of such faecal material can lead to a nasty infections in this area. Such a condition is most undesirable, painful and distressing to the bedridden wearer or to the infant.

Many articles exist on the market that allege the entrapment and immediate containment of faecal material in an effective manner. The prior art is rich with such examples. For instance, WO 96/19167 describes an absorbent article adapted to contain faecal material and highly fluidic material that is expelled at high velocity. The absorbent article comprises faecal containment members positioned transversely outward of the absorbent assembly, which function by interrupting the lateral movement of the faecal material. The faecal containment members are formed of resilient and porous materials to maintain and provide sufficient void volume to collect the faecal material, respectively. WO 94/14395 discloses a disposable absorbent article comprising a transverse partition disposed on the body facing surface of the topsheet and extending outwardly therefrom, to be upstanding and extend away from the plane of the disposable absorbent article. The transverse partition divides the absorbent article into a front portion and a rear portion, and presents an abrupt discontinuity between the two portions. Faecal material deposited in the rear portion is obstructed from longitudinally migrating to the front portion by the transverse partition. WO 97/01316 details a diaper with a bag for collecting faecal material and a urine collector for storing urine. The bag is formed by doubling over the diaper along a central imaginary line (perpendicular to the longitudinal sides of the diaper) and by drawing the edges (spaced apart from the central imaginary line) parallel to the imaginary line together. In such a manner, the faecal material is isolated from the skin of the wearer.

Japanese application JP 08-117 261 A discloses a diaper having a bag like structure, which is designed for incontinence sufferers. The bag comprises a slit or a hole in a position facing the anus with the slit or hole being surrounded by a surface coated with adhesive. The bag is formed of a water-repellent material. US 3 577 989 details a disposable plastic elimination-trapping bag for incontinence sufferers. The flange is specifically designed and shaped such that it is generally convex at its curved rearward end while being generally concave at its forward end. In particular, for a female patient, the convex portion of the flange is adapted to closely follow the curved body contour and to fit between the cheeks of the buttocks and below and around the anus while the concave forward end portion of the flange is designed to closely follow the curved contour of the body of the patient above the vulva. Between the end portions of the flange are elongated side portions dimensioned to closely follow the curved body flange are elongated side portions dimensioned to closely follow the curved body contour between the anus, cheeks and upper legs of the sufferer. EP 0 245 064 describes a faecal incontinence bag having flexible front and rear walls secured together around their periphery. The front wall has a hole for entry of the matter discharged by the wearer. The hole is surrounded by an adhesive pad of skin-compatible water-resistant material secured to the external surface of the front wall. The pad is generally heart-shaped so that when in position the concave portion of the heart-shaped pad is towards the front of the wearer. For all the prior art cases, the faecal matter is not entirely contained in the bag of the faecal management device. It is known that some of the faecal material seeps forward from the anal region and creeps into the urethra.

EP 0 753 290 A discloses a faecal collector with elastic attachement means. GB 1,092, 274 discloses a collector for usine specimens for use on female infants comprising a vedge-like projection.

Nonetheless, the need exists for a faecal management device that is extremely effective in completely isolating the faecal material from the sensitive urinary tract of the wearer. The present invention addresses this need by providing a projection at the front portion of the flange. It has been found that the presence of such a projection is uniquely advantageous and prevents the flow of faecal material out of the front portion of the flange towards the urinary tract. Furthermore, the presence of such a projection on the faecal management device causes no discomfort to the wearer, leads to a great reduction in infections and epidermal irritations derived from faecal material and results in a high level of wearer and carer satisfaction in relation to skin healthiness.

In another aspect of the present invention, the faecal management device with this projection can be advantageously used with a disposable diaper. The prior art is silent on such a combination. As described above, none of the prior art documents discloses two separate entities that work synergetically to isolate the faecal material from the sensitive organs of the wearer and which furthermore isolate the skin of the wearer from the absorbent material of the diaper.

### Summary of the Invention

A faecal management device constructed in accordance with the present invention comprises a bag having an aperture and an anatomically-shaped flange surrounding the aperture for adhesive attachment to the perianal area of longitudinal centreline and a transverse centreline wherein the transverse centreline segments the flange into a front portion and a rear portion.

In particular, the flange comprises a projection in the front portion. The projection is disposed between the outer periphery and the inner periphery of the flange in a direction parallel to the longitudinal direction. Preferably, the projection extends from the outer periphery to the inner periphery and is disposed in a symmetrical manner. The projection has an effective height ranging from 0.5 millimetres to 15 millimetres, preferably from 2 millimetres to 10 millimetres, more preferably an effective height of about 3 to 7 millimetres. The projection is particularly beneficial for female wearers where the projection is are adapted to fit snugly between the vulva and the anus, i.e., the perineum of the female wearer.

In another aspect of the present invention, the present faecal management device is used in combination with a disposable diaper.

### Brief description of the drawings

It is believed that the invention will be better understood from the foregoing description in conjunction with the accompanying drawings in which:
Figure 1 is a perspective view of a faecal management device according to the present invention;
Figure 2 shows a perspective view of the faecal management device in conjunction with a disposable diaper; and
Figure 3 is a partially cut-away perspective view of a disposable diaper embodying the present invention.

### Detailed description of the Invention

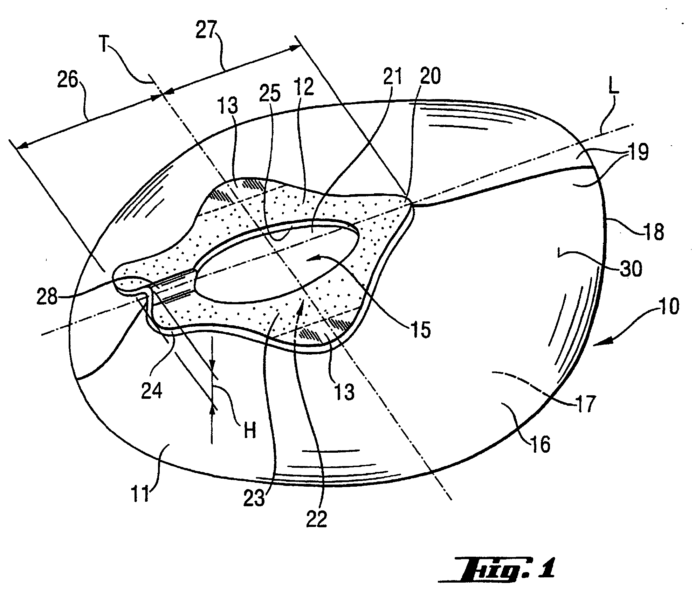

According to Figure 1, the faecal management device (10) of the present invention comprises a bag (11) having an aperture and an anatomically-shaped flange (12) surrounding the aperture (21) for adhesive attachment to the skin of the wearer in the anal region in between the buttocks of the wearer and in the gluteal groove.

From Figure 1, it is evident that the anatomically-shaped flange (12) comprises an outer periphery (24), an inner periphery (25) adjacent to and defining an aperture (21), a longitudinal centreline L and a transverse centreline T orthogonal thereto. The transverse centreline T segments the anatomically shaped flange (12) into a front portion (26) and a rear portion (27). The flange (12) further comprises a wearer facing surface (23) and a garment facing surface (22).

According to the present invention it has been surprisingly found that the presence of a projection positioned on the wearer facing surface of the anatomically shaped flange (12) is particularly effective in reducing and preventing any faecal matter from escaping from the device (10) between the skin and the flange. It is believed that the positioning of such a projection (28) at the front portion (26) of the wearer facing surface of the flange (23) not only provides a flange which more readily conforms to the contours of anatomy of the body, but also provides an improved seal at the positions on the flange, where the exit of faecal matter most readily occurs and thereby prevents any unnecessary contact between the skin and the excreted matter. In addition, a further surprising advantage of the present invention is that the presence of the front projection (28) also assists in the positioning of the device onto the desired skin location of the wearer of the device, further improving the sealing benefit. This is particularly useful for female wearers where the projection may be inserted into the urethra.

According to the present invention the projection is disposed between the outer periphery (24) and the inner periphery (25) of the anatomically-shaped flange (12) in a direction parallel to the longitudinal direction L. In addition the projection also extends in the transverse direction between the longitudinal side edges on either side of the longitudinal centreline L. More preferably, the flange (12) extends from the outer periphery (24) to the inner periphery (25). In the transverse direction, the projection (28) preferably does not extend across the entire transverse width of the flange but only up to 50% of the width more preferably up to 30% of the width of the flange measured through the centre of the projection.

The front projection (28) may be disposed symmetrically or asymmetrically about the longitudinal centreline L. In a more preferred embodiment, the projection (28) is disposed in a substantially symmetrical manner. According to the present invention the projection extends perpendicular to the plane of the flange. It is important that the projection (28) be upstanding and rise above the plane of the flange (12) to an effective height H sufficient to present an abrupt discontinuity to obstruct the movement of the faecal material. As used herein, the term "effective height" refers to the maximum distance in the Z-direction from the garment facing (23) of the flange (12) in its flat orientation of the projection, including adhesive if present on the projection surface. The projection (28) have an effective height ranging from 0.5 millimetres to 15 millimetres. Preferably, the projection (28) has an effective height ranging from 2 millimetres to 10 millimetres, more preferably an effective height of about 3 to 7 millimetres. The effective height measurements are carried out without the aid of a micrometer in order that no pressure is exerted onto the adhesive and flange material. Typically, the projection (28) is orthogonal to the plane of the flange (12). It should be recognised however that if the flange (12) has wrinkles, rugosities, undulations or other deviations from planarity, these should be taken into account at the position of the projection (28), when determining its effective height.

According to the present invention the projection (28) may have any shape. Typically the projection (28) has a longitudinal and or transverse substantially convex cross section. The front projection (28) is typically independently convex in shape and provides a hump-like or hill-like longitudinal cross section and or transverse cross section or provides a tubular cross section. The projection may also have a dual hump-like cross section, so as to provide a projection (28) having two or more distinct heights. Preferably, the hump exhibiting the maximum or effective height of the projection will be positioned towards the outer periphery (24) of the flange (12).

The projection (28) is preferably hollow or partially hollow to improve resiliency and flexibility of the projection but may also be completely filled. For embodiments wherein the projection (28) is hollow or partially hollow, the projection may require additional support means in order to maintain the desired resilient configuration of the projection (28). Suitable support means include adhesives or the provision of an elastic material connecting the longitudinal sides of the projection (28) to oneanother at least the base of the projection (28) on the wearer facing surface (23) of the flange (12).

According to the present invention the projection (28) should preferably be laterally compressible so that the projection will move inward when compressed by lateral forces rather than spring back. The projection (28) is resiliently deformable such that if for example the longitudinal sides are compressed the upper or top portion of the projection will be forced upwards and thereby provide a vertical extension of the projection in use and increase the overall effective height of the device. In this manner the sealing effect provided by the projection (28) and more preferably in combination with the flange (12) and adhesive (20) is always maintained, even when the wearer of the device is active during use and thereby places increased pressure onto the device.

The projection (28) configuration may be selected in order to provide further improved fit for example if intended for a particular wearer group. For example the front projection (28) for a device intended for female wearers should typically have a greater effective height than the front projection (28) for a device intended for male wearers. However the exact design and dimension can readily be selected by the skilled person in the field.

The projection (28) may be formed as an integral part of the flange or may be provided as separate entities whereby a material which may be different or identical to the flange material is attached to the flange (12) using means known in the art, typically adhesive. Preferably however, the projection (28) is formed as an integral part of the flange. The projection (28) may be made by forming a single pleat in the constituent material of the flange (12) or by thermobonding the flange material. Alternatively, the projection (28) may be provided by a body compatible adhesive material. In a preferred embodiment of the present invention however, the projection (28) is formed from the flange material itself and utilises an adhesive support means.

The projection (28), may either be coated with adhesive or be substantially free from adhesive. According to a preferred embodiment of the present invention, the projection (28) is also covered with a body-compatible adhesive. For embodiments wherein the projection (28) comprises a dual hump longitudinal cross section for example, it has been found particularly beneficial to provide only one of the surfaces of the hump, preferably the hump providing "the effective height" with a body compatible adhesive. The remaining hump surface may be provided with an anti slip material such as rubber.

As shown in Figure 1, the aperture (21) is surrounded by a flange (12) and may be provided in any shape or size, such as circular, oblong, heart shaped and may be symmetrical or asymmetrical, preferably the aperture has an oblong configuration either in the longitudinal or in the transversal direction, most preferably the contours of the aperture are in the shape of two ellipses with the respective main axes being substantially perpendicular.

The flange (12) is attached to the bag (11) according to any means known to the man skilled in the art which may provide permanent or releasable attachment. Preferably however, the flange is attached to the bag by adhesive. Typically, the bag will be attached to the flange, towards the outer periphery of flange so as not to cause any obstruction for the entering faecal matter.

The flange may be provided in any size depending on the wearer group for which the device is intended. Similarly the flange may be provided in any shape and preferably has a symmetrical shape preferably comprising a plurality of lobes (13). The flange comprises a garment facing portion (22) and a wearer facing portion (23).

The flange (12) should be made of soft, flexible and malleable material to allow easy placement of the flange to the perianal area. Typical materials include nonwoven materials, wovens, open celled thermoplastic foams, closed-cell thermoplastic foams, composites of open celled foams and stretch nonwoven, and films, air-laid materials including natural and synthetic fibres, thermally bonded airlaid materials, felt fabrics, needlepunched fabrics, spunlaced fabrics, fluid jet entangled fabrics, wet-laid fabrics, dry-laid fabrics, melt-blown fabrics, staple fibre carding fabrics, spunbonded fabrics, stitch-bonded fabrics, apertured fabrics, combinations of the above or the like. A closed-cell foam of polyethylene has been found effective, but more preferably an open celled polyurethane foam is used. Preferably, such foams have a thickness within the general range of 0.1 to 5 millimetres and a density of 5 to 250 g/m², more preferably 50 g/m². Other thermoplastic foam materials, or other suitable plastics sheet materials having the described properties of such foams (i.e., softness, pliability, stretchability, and contractability) might also be used. Preferably, the material of garment facing surface (23) of the flange (12) may extend into the defined aperture (21) area so as to form a skirt or flap of material which prevents unintentional adhesion of the surface edges of the flange defining the aperture to oneanother during use.

According to the present invention the faecal management device (10) further comprises an attachment means to secure the device to the wearer. Such means include straps and more preferably comprises a body-compatible pressure sensitive adhesive (20) applied to the wearer facing portion (23) of the flange (12).

The adhesive (20) is preferably covered with a release means (not shown) in order to protect the adhesive (20), such as siliconized paper. The adhesive (20) can cover the entire wearer facing surface of the flange or more preferably have at least one, preferably two to six non-adhesive portions. These portions may be adhesive free or may contain inactivated or covered adhesives. As is evident from Figure 1, the adhesive is in one preferred embodiment not applied to the entire wearer facing surface area of the flange (12), so as to provide lobes (13) on either side of the flange (12) which are non-adhesive and can thereby serve to facilitate placement and removal of the device whilst avoiding contact with the adhesive. These lobes are however preferably also covered by the release means. Before application of the faecal management device (10) to the skin of the wearer, the release means if present is removed.

According to the present invention any medically approved water resistant pressure sensitive adhesive may be used to attach the device to the perianal area of the wearer, such as hydrocolloid adhesives and hydrogel adhesives. Particularly effective adhesives in providing the desired adhesive properties to secure the flange to the skin of the wearer at the sensitive perianal area, whilst allowing for relatively painless application and removal are formed from crosslinking polymers with a plastisicer to form a 3-dimensional matrix.

The adhesive (20) can be applied to the wearer facing surface of the flange (12) by any means known in the art such as slot coating, spiral, or bead application or printing. Typically the adhesive is applied at a basis weight of from 20g/m² to 2500g/m², more preferably from 500g/m² to 2000g/m² most preferably from 700g/m² to 1500g/m² depending on the end use envisioned. For example for faecal management devices to be used for babies the amount of adhesive may be less than for faecal management devices designed for active adult incontinence sufferers.

The bag (11) as used herein is a flexible receptacle for the containment of excreted faecal matter. The bag (11) can be provided in any shape or size depending on the intended use thereof, i.e. whether the device is intended for bedridden patients or active patients suffering from incontinence or requiring an artificial bowel or for infants. For example elongated bags which are principally tubular or rectangular are typically utilised by bedridden patients and elderly incontinence sufferers. For more active wearers whether infants or adults, the faecal management device should preferably be anatomically shaped such that the device follows the contours of the body and can be worn inconspicuously by the wearer under normal garments.

Particularly, preferred shapes are flat circular type bags, cone shaped bags, truncated cone shaped bags and pyramidal or truncated pyramidal shaped bags. In a most preferred embodiment of the present invention, the bag (11) has a substantially truncated cone shape. Typically the bags will have a wearer facing portion (16) and a garment facing portion (17). The wearer facing portion (16) of the faecal management device (10) is disposed adjacent the buttocks of the wearer. As such, the wearer facing portion (16) amply covers the buttocks of the wearer and does not hang between the thighs of the wearer.

In addition, the bag (11) is preferably shaped to allow at least partial insertion and retention of the bag in-between the buttocks of the wearer and thereby ensure good contact between the flange and the skin of the wearer. For example the bag (11) may be provided with a neck portion or conduit.

The bag (11) is preferably designed to provide sufficient volume for faecal material under a variety of wearing conditions, also when worn by a freely moving, i.e. not bedridden wearer. Sitting on the bag (11), for example, will result in a largely reduced volume in some areas of the bag. Thus, the bag (11) is preferably shaped to provide sufficient volume in areas which are not subjected to much pressure in wearing conditions such as sitting.

The bag (11) is designed to safely contain any entrapped material, typically it will be liquid impermeable, yet it may be breathable. The bag is designed of sufficient strength to withstand rupture in use, also when pressure on the bag is exerted in typical wearing conditions, such as sitting.

According to the present invention, depending on the shape of the bag (11) required, the bag (11) may be provided from a unitary piece of material or from a number of separate pieces of material, which may be identical or different and which are sealed at their respective peripheries.

In one preferred embodiment the bags herein have a wearer facing portion (16) and a garment facing portion (17) which comprise separate pieces of material. The wearer facing portion (16) and the garment facing portion (17) are sealed at the periphery of the bag (11), thus creating a bag peripheral rim (18). As is visible from Figure 1, the wearer facing portion (16) of the bag (11) may comprise two further sections (19), which are secured to each other by means known to the man skilled in the art, such as adhesive, thermobonding or pressure bonding in order to provide the desired bag configuration. Said rim (18) may also be inside the bag, thus being coextensive with the inner surface (15) of the bag (11) rather than with the outer surface (30) of the bag (11). Preferably the bag (11) is asymmetrical to the transversal axis, so that the distance measured in the longitudinal direction from the centre of the aperture (21) to the front end of the bag (11) is shorter than the distance measured to the rear end of the bag (11).

According to the present invention the bag (11) can comprise one or multiple layers, preferably two or three layers. The layer on the inside of the bag (11), which will typically at least partially come in contact with faecal material is called the inner layer. The outermost layer of the bag, which will typically at least partially come in contact with the skin to the wearer and the garments of the wearer, is called the outer layer.

The layers of the bag material may be provided from any material, preferably so that the bag is liquid impervious. The layers may in particular comprise any material such as non-wovens or films. In a preferred embodiment of the present invention a laminate may be formed from a non-woven layer and a film. The laminate can be formed by means known to the man skilled in the art.

Any non-woven layer can comprise felt fabrics, spunlaced fabrics, fluid jet entangled fabrics, air-laid fabrics, wet-laid fabrics, dry-laid fabrics, melt-blown fabrics, staple fibre carding fabrics, spunbonded fabrics, stitch-bonded fabrics, apertured fabrics, combinations of the above or the like.

Suitable film materials for any of said layers preferably comprise a thermoplastic material. The thermoplastic material can be selected from among all types of hot-melt adhesives, polyolefins especially polyethylene, polypropylene, amorphous polyolefins, and the like; material containing meltable components comprising fibres or polymeric binders including natural fibres such as cellulose - wood pulp, cotton, jute, hemp; synthetic fibres such as fibreglass, rayon, polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., III, US under the designation EXXAIRE or those supplied by Mitsui Toatsu Co., Japan under the designation ESPOIR NO; and monolithic breathable materials such as Hytrel™ available from DuPont and Pebax™ available from ELF Atochem, France.

In a preferred embodiment a film, which is comprised in any layer, is preferably permeable to gases such as air and to vapour such as water vapour in order to avoid the problem of entrapment and condensation of moisture vapour given off by the body of the wearer and thus, the hot, clammy and uncomfortable conditions after a short period of use.

The outer layer of the bag is preferably provided with a non-woven layer. Such material layers present an uneven surface to the skin of the wearer and thus reduce significantly the problem of occlusion and greatly improve skin healthiness.

In one preferred embodiment of the present invention the bag comprises two layers. Preferably the outer layer comprises a non-woven layer and the inner layer comprises a film.

In yet another preferred embodiment of the present invention, the bag (11) comprises three layers, preferably one film and two non-woven layers. In an even more preferable embodiment the film is interposed between the two non-woven layers. This sequence of layers results in a closed fibrous structure, which has a particularly pleasing sensation on contact with the skin of the wearer. In yet another preferred embodiment the inner layer comprises a film and the other two layers comprise non-wovens.

The non-woven layer or the non-woven layers comprised by the bag (11) may be hydrophobic or hydrophilic. If the bag (11) does not comprise a film layer, preferably at least one non-woven layer is hydrophobic. As a consequence, fluid penetration is resisted through the wearer facing portion (16) and the garment facing portion (17) of the faecal management device (10). If the bag comprises a film or a hydrophobic non-woven layer, further non-woven layers may be hydrophilic.

Typically, the non-woven layer is treated with a surface active material, such as a fluorchemical or other hydrophobic finishings, to provide the requisite hydrophobicity. The non-woven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example.

The non-woven layer can also be treated with agents to improve the tactile perceivable softness of the wearer facing portion (16) and the garment facing portion (17). The agents include but are not limited to vegetable, animal or synthetic oils, silicone oils and the like. The presence of these agents are known to impart a silky or flannel-like feel to the non-woven layer without rendering it greasy or oily to the tactile sense of the wearer. Additionally, surfactant material, including anionic, non-anionic, cationic and non-cationic surfactants, may be added to further enhance softness and surface smoothness.

Furthermore, the non-woven layer may be impregnated with a lotion to provide desirable therapeutic or protective coating lotion benefits. The lotion coating on the wearer facing portion (16) and the garment facing portion (17) is transferable to the skin of the wearer by normal contact and wearer motion and/or body heat. Generally, mineral oil in the form of a lotion is recognised as being effective in imparting a soothing, protective coating to the skin of the wearer. It is also possible to impregnate the non-woven layer with a solid oil phase of cream formulation or to incorporate into the non-woven layer an array of pressure- or thermal- or hydrorupturable capsules containing for example, baby oil.

In one embodiment of the present invention the bag may contain absorbent material. The absorbent material may comprise any absorbent material which is capable of absorbing and retaining liquids. The absorbent material may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The absorbent material may be positioned in the bag (11) in any suitable manner. For example, the absorbent material may be loosely arranged within the bag or may be secured to the inner layer of the bag (11). Any known techniques for securing absorbent material to nonwoven and film substrates may be used to secure the absorbent material to the inner layer of the bag. The absorbent material may also be arranged to have any desired shape or configuration (e.g., rectangular, oval, circular, etc.).

### Detailed description of a diaper to be worn in combination with the faecal management device

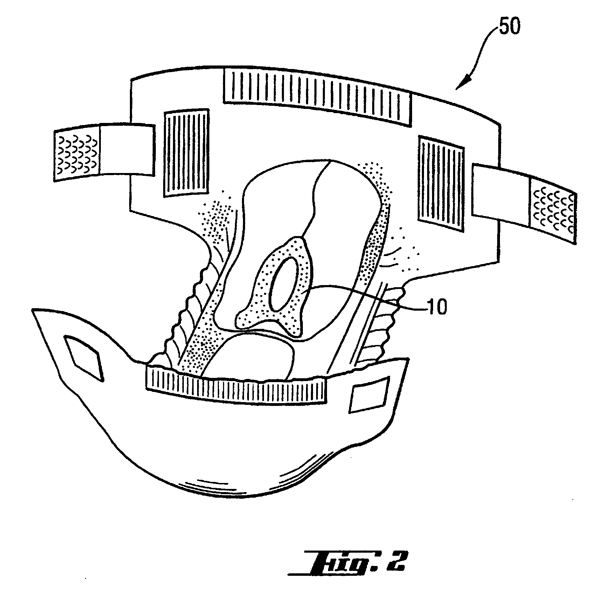

The faecal management device (10) of the present invention has been found to be particularly useful and beneficial when used in conjunction with a garment, or diaper (50), preferably a disposable diaper - refer to Figure 2. The faecal management device (10) is preferably first positioned in the perianal area of the wearer before the disposable diaper (50) is applied. in particular, the diaper (50) is positioned over the faecal management device (10) and fastened in a conventional manner around the body of the wearer. It has been found that, in addition, to providing excellent separation between urine and faecal material, the combined faecal management device (10) and diaper (50) system actually reduces skin irritation, which may at times occur, especially since the group of typical wearers includes the very old, the very young and the unhealthy wearers. In effect, the presence of the faecal management device (10) permits the formation of a separation layer between the skin of the wearer and the diaper (50), i.e. a part of the absorbent core (58) of the diaper (10). The diaper (50) can be of the conventional type (an embodiment of which is described below although not a limiting example by any means) or can be adapted to contain in an effective and comfortable manner the faecal management device (10) according to the teachings of the present invention.

As used herein, the term "disposable diapers" refers to articles which absorb and contain body extrudates; and more specifically, refers to articles which are placed against or in proximity to the body of the wearer to absorb and contain the various extrudates discharged from the body and which are intended to be discarded after a single use (i.e., they are not intended to be laundered or otherwise restored or reused) and, preferably, to be recycled, composted or otherwise disposed of in an environmentally compatible manner. As used herein, the term "diaper" refers to a garment generally worn by infants or incontinence sufferers that is drawn up between the legs and fastened about the waist of the wearer.

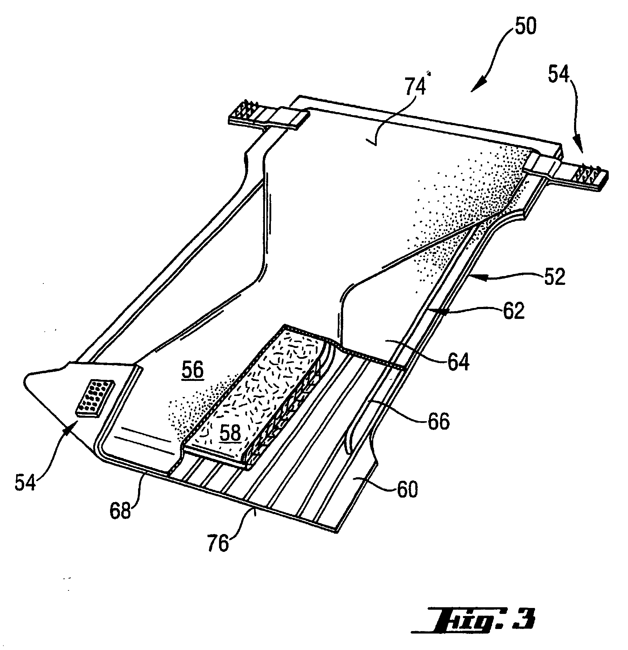

Figure 3 is a partially cut-away perspective view of a diaper (50) embodying the present invention prior to it being placed on the wearer over the faecal management device (10). As is visible from Figure 3, a preferred diaper (50) comprises a body portion (52) and a refastenable mechanical fastening device (54). A preferred body portion (52) comprises a liquid pervious topsheet (56), and absorbent core (58), a liquid impervious backsheet (60), and elastically contractible leg cuffs (62); each leg cuff (62) preferably comprising a side flap (64) and one or more elastic members (66). For simplicity purposes, only one elastic member (66) is shown in the side flap (64). While the topsheet (56), the absorbent core (58), the backsheet (60), the side flaps (64), and the elastic members (66) may be assembled in a variety of well-known configurations. A preferred disposable diaper configuration is shown and generally described in US 3,860,003, an even more preferred disposable diaper configuration is shown and generally described in WO 93/16669. In this preferred diaper configuration, the backsheet (60) is joined to the topsheet (56); the absorbent core (58) is positioned between the topsheet (56) and the backsheet (60); the side flaps (64) extend outwardly from and along each side edge of the absorbent core (58); and the elastic member (66) is operatively associated with each side flap (64).

Figure 3 shows the body portion (52) in which the topsheet (56) and the backsheet (60) are coextensive and have length and width dimensions generally larger than those of the absorbent core (58). The topsheet (56) is superposed on the backsheet (60) thereby forming the periphery (68) of the body portion (52).

The body portion (52) has an inside surface (74) and an outside surface (76). When a backsheet (60) is used, it typically forms the outside surface (76) of the body portion (52). The inside surface (74) is that surface of the diaper (50) opposite the outside surface (76) and in the embodiment shown is typically formed by the topsheet (56). In general, the inside surface (74) of the diaper (50) is that surface coextensive with the outside surface (76) and which is for the greater part in contact with the wearer when the diaper (50) is worn.

The absorbent core (58) of the body portion (52) may be any absorbent means which is generally compressible, conformable, non-irritating to the skin of the wearer, and capable of absorbing and retaining liquids such as urine and other certain bodily discharges. The absorbent core (58) may be manufactured in a variety of sizes and shapes (for example, rectangular, hour-glass, "T"-shaped, asymmetric, etc.) and from a wide variety of liquid absorbent materials commonly used in disposable diapers and other absorbent articles such as comminuted wood pulp which is generally referred to as airfelt. Examples of other suitable absorbent materials include creped cellulose wadding, meltblown polymers including coform, crosslinked cellulosic fibers, tissue including tissue wraps, absorbent foams, absorbent sponges, superabsorbent polymers, absorbent gelling materials, or any equivalent materials or combinations of materials. The configuration and construction of the absorbent core (58) may also be varied (for example, the absorbent core (58) may have varying caliper zones, hydrophilic gradients, superabsorbent gradients, or lower average density and lower average basis weight acquisition zones; or may comprise one or more layers or structures). Further, the size and absorbent capacity of the absorbent core (58) may be varied to accommodate wearers ranging from infants to adults.

The backsheet (60) is impervious to liquids (for example, urine) and is preferably manufactured from a thin plastic film, preferably a thermoplastic film, although other flexible liquid impervious materials may also be used. As used herein, the term "flexible" refers to materials which are compliant and which will readily conform to the general shape and contours of the human body. The backsheet (60) prevents the exudates absorbed and contained in the absorbent core (58) from soiling articles which are in contact with the diaper (50) such as undergarments and bedding. The backsheet (60) may thus comprise polymeric films such as thermoplastic films of polyethylene or polypropylene, or composite materials such as film-coated non-woven material. Exemplary films are manufactured by Tredegar Industries, Inc. of Terre Haute, Ind., USA or BP-Chemical PlasTec, Rotbuchenstrasse 1, D-8000 Munchen, Germany.

The backsheet (60) is preferably textured to provide a more clothlike appearance. Further, the backsheet (60) may also permit vapours to escape from the absorbent core (58) while still preventing exudates from passing through the backsheet (60) by, for example, being supplied with microapertures. The size of the backsheet (60) is dictated by the size of the absorbent core (58) and the exact diaper design selected.

The topsheet (56) of the diaper is compliant, soft feeling and non-irritating to the skin of the wearer. Further, the topsheet (56) is liquid pervious permitting liquids (for example, urine) to readily penetrate through its thickness. A suitable topsheet (56) may be manufactured from a wide range of materials, such as porous foams, reticulated foams, apertured films; or woven or non-woven webs of natural fibres (for example, wood or cotton fibres) or from a combination of natural and synthetic fibres. Preferably, it is made of a material that isolates the skin of the wearer from liquids retained in the absorbent core (58).

There are a number of manufacturing techniques which may be used to manufacture the topsheet (56). For example, the topsheet (56) may be a non-woven web of fibres. An exemplary topsheet (56) is carded and thermally bonded by means well-known to those skilled in the fabric art. A suitable topsheet (56) is manufactured by, for example, Veratec Inc., a division of International Paper Company, of Walpole, Mass., USA. A topsheet (56) particularly preferred for incontinence garments comprises a formed thermoplastic film.

## Claims

1. A faecal management device (10) comprising a bag (11), said bag (11) having an aperture and an anatomically-shaped flange (12) surrounding said aperture for adhesive attachment to the perianal area of wearer, said anatomically-shaped flange (12) being attached to said bag (11) wherein said anatomically-shaped flange comprises an outer periphery (24), and an inner periphery (25) adjacent said aperture, a longitudinal centreline (L) and a transverse centreline (T) orthogonal thereto, said transverse centreline (T) segmenting said anatomically-shaped flange (12) into a front portion (26) and a rear portion (27), said flange having a wearer facing surface (23) and a garment facing surface (22), **characterised in that**
said wearer facing surface (23) of said anatomically-shaped flange (12) comprises a resiliently deformable projection (28) in said front portion (26) and **in that** said projection (28) is disposed between said outer periphery (24) and said inner periphery (25) of said anatomically-shaped flange (12) in a direction parallel to said longitudinal direction (L) and said projection (28) has an effective height ranging from 0.5 millimetres to 15 millimetres.

2. A faecal management device (10) according to claim 2 wherein said projection (28) extends from said outer periphery (24) to said inner periphery (25).

3. A faecal management device (10) according to any of the preceding claims wherein said projection (28) is symmetrical about said longitudinal axis.

4. A faecal management device (10) according to any of the preceding claims wherein said projection (28) has an effective height ranging from 2 millimetres to 10 millimetres, preferably from about 3 millimeters to 7 millimetres.

5. A faecal management device (10) according to any of the preceding. claims wherein said projection (28) is adapted to fit between the vulva and the anus of a female wearer.

6. The use of a faecal management device (10) according to any of the preceding claims in combination with a disposable diaper (50).

7. The use of a faecal management device (10) according to claim 6, whereby said faecal management device (10) is first positioned in the perianal area of a female wearer, said projection (28) being fitted between the vulva and anus and then said disposable diaper (50) is positioned over said faecal management device (10) and fastened in a conventional manner around body of said female wearer.

8. The use of a faecal management device (10) according to claim 6, wherein said faecal management device (10) provides a separation layer between skin of said wearer and said disposable diaper (50).

9. The use of a faecal management device (10) according to claim 7 wherein said projection (28) prevents flow of faecal matter into the urethra of a female wearer.

## Patentansprüche

1. Fäkalienhandhabungsvorrichtung (10) mit einem Beutel (11), der Beutel (11) weist eine Öffnung und einen anatomisch geformten Flansch (12) auf, der die Öffnung zum klebenden Befestigen am Perianalbereich eines Trägers umgibt, der anatomisch geformte Flansch (12) ist an dem Beutel (11) befestigt, wobei der anatomisch geformte Flansch einen äußeren Rand (24) und einen inneren Rand (25) benachbart zur Öffnung, eine longitudinale Mittellinie (L) und eine transversale Mittelline (T) umfaßt, die transversale Mittellinie (T) unterteilt den anatomisch geformten Flansch (12) in einen vorderen Teil (26) und einen hinteren Teil (27), der Flansch weist eine einem Träger zugewandte Oberfläche (23) und eine der Kleidung zugewandte Oberfläche (22) auf, **dadurch gekennzeichnet, daß** die dem Träger zugewandte Oberfläche (23) des anatomisch geformten Flansches (12) einen elastisch verformbaren Vorsprung (28) im vorderen Teil (26) umfaßt, und daß der Vorsprung (28) zwischen dem äußeren Rand (24) und dem inneren Rand (25) des anatomisch geformten Flansches (12) in einer Richtung parallel zur Längsrichtung (L) vorgesehen ist, und der Vorsprung (28) eine effektive Höhe im Bereich von 0,5 mm bis 15 mm besitzt.

2. Fäkalienhandhabungsvorrichtung (10) nach Anspruch 1, wobei der Vorsprung (28) sich vom äußeren Rand (24) bis zum inneren Rand (25) erstreckt.

3. Fäkalienhandhabungsvorrichtung (10) nach einem der vorstehenden Ansprüche, wobei der Vorsprung (28) symmetrisch zur Längsachse ist.

4. Fäkalienhandhabungsvorrichtung (10) nach einem der vorstehenden Ansprüche, wobei der Vorsprung (28) eine effektive Höhe im Bereich von 2 mm bis 10 mm, vorzugsweise von etwa 3 mm bis 7 mm, aufweist.

5. Fäkalienhandhabungsvorrichtung (10) nach einem der vorstehenden Ansprüche, wobei der Vorsprung (28) angepaßt ist, um zwischen die Vulva und den Anus einer weiblichen Trägerin zu passen.

6. Verwendung einer Fäkalienhandhabungsvorrichtung (10) nach einem der vorstehenden Ansprüche in Verbindung mit einer Wegwerfwindel (50).

7. Verwendung einer Fäkalienhandhabungsvorrichtung (10) nach Anspruch 6, wobei die Fäkalienhandhabungsvorrichtung (10) zuerst im Perianalbereich einer weiblichen Trägerin angeordnet wird, der Vorsprung (28) zwischen Vulva und Anus eingepaßt wird und dann die Wegwerfwindel (50) über der Fäkalienhandhabungsvorrichtung (10) angeordnet und in einer herkömmlichen Weise um den Körper der weiblichen Trägerin herum befestigt wird.

8. Verwendung einer Fäkalienhandhabungsvorrichtung (10) nach Anspruch 6, wobei die Fäkalienhandhabungsvorrichtung (10) eine Trennlage zwischen der Haut der Trägerin und der Wegwerfwindel (50) bildet.

9. Verwendung einer Fäkalienhandhabungsvorrichtung (10) nach Anspruch 7, wobei der Vorsprung (28) verhindert, daß fäkales Material in die Harnröhre einer weiblichen Trägerin fließt.

## Revendications

1. Dispositif de prise en charge des matières fécales (10) comprenant un sac (11), ledit sac (11) ayant une ouverture et une collerette de forme anatomique (12) entourant ladite ouverture pour une fixation adhésive à la zone périanale de l'utilisateur, ladite collerette de forme anatomique (12) étant fixée audit sac (11), dans lequel ladite collerette de forme anatomique comprend une périphérie extérieure (24) et une périphérie intérieure (25) près de ladite ouverture, une ligne médiane longitudinale (L) et une ligne médiane transversale (T) perpendiculaire à celle-ci, ladite ligne médiane transversale (T) segmentant ladite collerette de forme anatomique (12) selon une partie avant (26) et une partie arrière (27), ladite collerette ayant une surface tournée vers l'utilisateur (23) et une surface (22) tournée vers les vêtements, **caractérisé en ce que** ladite surface tournée vers l'utilisateur (23) de ladite collerette de forme anatomique (12) comprend un prolongement élastiquement déformable (28) dans ladite partie avant (26) et **en ce que** ledit prolongement (28) est disposé entre ladite périphérie extérieure (24) et ladite périphérie intérieure (25) de ladite collerette de forme anatomique (12) dans une direction parallèle à ladite direction longitudinale (L) et ledit prolongement (28) présente une hauteur efficace se situant dans la gamme de 0,5 mm à 15 mm.

2. Dispositif de prise en charge des matières fécales (10) selon la revendication 1, dans lequel ledit prolongement (28) s'étend depuis ladite périphérie extérieure (24) à ladite périphérie intérieure (25).

3. Dispositif de prise en charge des matières fécales (10) selon l'une quelconque des revendications précédentes, dans lequel ledit prolongement (28) est symétrique autour dudit axe longitudinal.

4. Dispositif de prise en charge des matières fécales (10) selon l'une quelconque des revendications précédentes, dans lequel ledit prolongement (28) a une hauteur efficace se situant dans la gamme de 2 mm à 10 mm, de préférence d'environ 3 mm à 7 mm.

5. Dispositif de prise en charge des matières fécales (10) selon l'une quelconque des revendications précédentes, dans lequel ledit prolongement (28) est agencé pour s'adapter entre la vulve et l'anus d'une personne de sexe féminin.

6. Utilisation d'un dispositif de prise en charge des matières fécales (10) selon l'une quelconque des revendications précédentes en combinaison avec une couche jetable (50).

7. Utilisation d'un dispositif de prise en charge des matières fécales (10) selon la revendication 6, dans laquelle ledit dispositif de prise en charge des matières fécales (10) est dans une première position dans la zone péri-anale d'une personne de sexe féminin, ledit prolongement (28) étant adapté entre la vulve et l'anus et ensuite ladite couche jetable (50) est positionnée sur ledit dispositif de prise en charge des matières fécales (10) et fixée d'une manière classique autour du corps de ladite personne de sexe féminin.

8. Utilisation d'un dispositif de prise en charge des matières fécales (10) selon la revendication 6, dans laquelle ledit dispositif de prise en charge des matières fécales (10) fournit une couche de séparation entre la peau de ladite personne et ladite couche jetable (50).

9. Utilisation d'un dispositif de prise en charge des matières fécales (10) selon la revendication 7, dans laquelle ledit prolongement (28) empêche l'écoulement des matières fécales dans l'urètre d'une personne de sexe féminin.
